# EUROPEAN PATENT SPECIFICATION

(11) **EP 2 356 680 B1**
(45) Date of publication and mention of the grant of the patent: **08.04.2015**
(21) Application number: 09826745.3
(22) Date of filing: 12.11.2009
(51) Int. Cl.: H01L 23/535, H01L 23/498

(54) **EXTREMELY STRETCHABLE ELECTRONICS**
EXTREM DEHNBARE ELEKTRONIK
DISPOSITIFS ELECTRONIQUES EXTREMEMENT ETIRABLES

(30) Priority: 07.10.2009 US 575008; 12.11.2008 US 113622 P
(43) Date of publication of application: 17.08.2011
(60) Divisional application: 15157469.6
(73) Proprietor: Mc10, Inc., Waltham, MA 02451 (US)
(72) Inventor: ARORA, William, J., Boston MA 02116 (US); GHAFFARI, Roozbeh, Cambridge MA 02142 (US)
(74) Representative: Driver, Virginia Rozanne
(86) International application number: PCT/US2009/064199
(87) International publication number: WO 2010/056857

(56) References cited:
- WO-A2-03/021679
- WO-A2-2004/095536
- US-A1- 2002 094 701
- US-B1- 6 360 615
- US-B1- 6 613 979
- US-B2- 6 743 982

## Description

### CROSS-REFERENCE TO RELATED APPLICATIONS

This application claims the benefit of United States Provisional Application No. 61/113,622 entitled "Extremely Stretchable Interconnects" filed on November 12, 2008. Also, this application is a continuation-in-part of, and claims the benefit of United States Non-Provisional Application No. 12/575,008, entitled "Catheter Balloon Having Stretchable Integrated Circuitry and Sensor Array" filed on October 7, 2009. Application No. 12/575,008 claimed the priority of United States Provisional Application Nos. 61/103,361, filed October 7, 2008 and 61/113,007, filed November 10, 2008.

### FIELD OF THE INVENTION

The present invention relates to systems, apparatuses, and methods utilizing expandable or stretchable integrated circuitry, and more particularly to extremely stretchable integrated circuitry.

### BACKGROUND OF THE INVENTION

The field of stretchable electronics continues to grow due to the demand of high performance and mechanically unconstrained applications of the future. Stretchable interconnects between integrated circuits are described for example in International Patent Application WO03021679. However, stretchable electronics have been thus far limited in stretchability. This has limited the ability of stretchable electronics to accommodate applications that require more extreme stretchability. Therefore a need exists for extremely stretchable electronics.

### SUMMARY OF THE INVENTION

The invention is defined in claim 1, additional features in claims 2-15. This invention is for extremely stretchable electrical interconnects and methods of making the same. In embodiments, the invention comprises a method of making stretchable electronics, which in some embodiments can be out of high quality single crystal semiconductor materials or other semiconductor materials, that are typically rigid. For example, single crystal semiconductor materials are brittle and cannot typically withstand strains of greater than about +/- 2%. This invention describes a method of electronics that are capable of stretching and compressing while withstanding high translational strains, such as in the range of -100,000% to +100,000%, and/or high rotational strains, such as to an extent greater than 180°, while maintaining electrical performance found in their unstrained state.

In embodiments, the stretching and compressing may be accomplished by fabricating integrated circuits (ICs) out of thin membrane single crystal semiconductors, which are formed into "islands" that are mechanically and electrically connected by "interconnects," and transferring said ICs onto an elastomeric substrate capable of stretching and compressing. The islands are regions of non-stretchable/compressible ICs, while the interconnects are regions of material formed in a way to be highly stretchable/compressible. The underlying elastomeric substrate is much more compliant than the islands, so that minimal strain is transferred into the islands while the majority of the strain is transferred to the interconnects, which only contain electrical connections and not ICs. Each interconnect attaches one island to another island, and is capable of accommodating strain between the two aforementioned islands, including translation, rotation, or a combination of translation with rotation of one island relative to another. Even though the interconnects may be made of a rigid material, they act like weak springs rather than rigid plates or beams. This configuration thereby allows for the making of extremely stretchable electronics.

These and other systems, methods, objects, features, and advantages of the present invention will be apparent to those skilled in the art from the following detailed description of the preferred embodiment and the drawings.

### BRIEF DESCRIPTION OF THE DRAWINGS

The invention and the following detailed description of certain embodiments thereof may be understood by reference to the following figures:
Figure 1 depicts an overhead view of an embodiment of the present invention showing two device islands connected edge-to-edge by a monolithically formed extremely stretchable interconnect, prior to being stretched.
Figure 2 depicts an overhead view of an embodiment of the present invention showing two device islands connected edge-to-edge by two extremely stretchable interconnects.
Figure 3 depicts an overhead view of an embodiment of the present invention showing two device islands connected edge-to-edge by three extremely stretchable interconnects; in this case, the long bars of the interconnects are rotated by 90° which allows them to be longer than if they were not rotated.
Figure 4 depicts four device islands arranged in a square matrix in an embodiment of the present invention, with each edge connected by an extremely stretchable interconnect to its nearest neighbors island edge, and the interconnects are formed so as to maximize the amount of chip area that is used for either an island or interconnect.
Figure 5 depicts the case of Figure 1, with the short bars widened for extra mechanical strength at those locations.

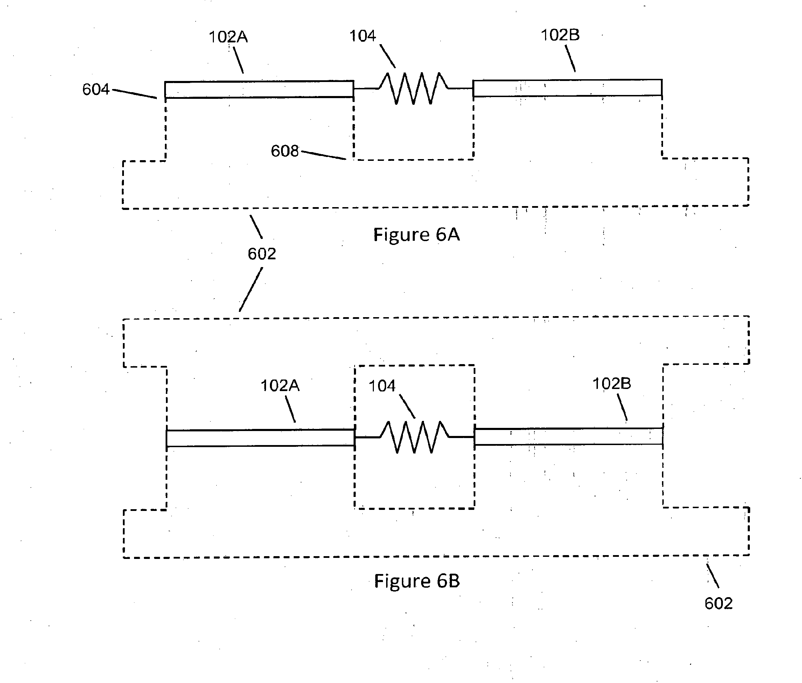

Figure 6 depicts embodiments of the present invention, where (a) is a side view of device islands and extremely stretchable interconnects transferred onto an elastomeric substrate. In this case, the substrate has been molded to have posts that are of the same area as the device islands (note that in embodiments these could be smaller or larger than the device islands). The height "h" of the molded post regions may range from, but is not limited to, about 1-1000 µm. The interconnects are located in between these regions as shown (b) Side view as before, with a similarly shaped elastomeric superstrate to serve as an encapsulation layer protecting the devices from direct mechanical contact.
Figure 7 depicts a side view of a two-layer PDMS substrate in an embodiment of the present invention comprising silicon device islands adhered to top layer, free-standing interconnects, and square wave ripples in the lower layer PDMS to promote increased stretching through the substrate.
Figure 8 depicts an embodiment of the present invention with a side view of two layers of cured photoresist (SU-8 50 and SU-8 2002) used to make the two-layer PDMS substrate described in Figure 7.
Figure 9 depicts an embodiment of the present invention with a side view of a two-layer PDMS substrate consisting of sinusoidal waves in the lower layer of PDMS to promote increased stretching through the substrate.

While the invention has been described in connection with certain preferred embodiments, other embodiments would be understood by one of ordinary skill in the art and are encompassed herein.

### DETAILED DESCRIPTION OF THE INVENTION

The present invention accomplishes extremely stretchable electronics by forming the electronics on discrete islands 102 of silicon.

With reference to the present invention, the term "stretchable", and roots and derivations thereof, when used to modify circuitry or components thereof is meant to encompass circuitry that comprises components having soft or elastic properties capable of being made longer or wider without tearing or breaking, and it is also meant to encompass circuitry having components (whether or not the components themselves are individually stretchable as stated above) that are configured in such a way so as to accommodate and remain functional when applied to a stretchable, inflatable, or otherwise expandable surface. The term "expandable", and roots and derivations thereof, when used to modify circuitry or components thereof is also meant to have the meaning ascribed above. Thus, "stretch" and "expand", and all derivations thereof, may be used interchangeably when referring to the present invention.

In embodiments, the discrete islands mention above are discrete operative (in embodiments, arranged in a "device island" arrangement) and are themselves capable of performing the functionality described herein, or portions thereof. In embodiments, such functionality of the operative devices can include integrated circuits, physical sensors (e.g. temperature, pH, light, radiation etc), biological and/or chemical sensors, amplifiers, A/D and D/A converters, optical collectors, electro-mechanical transducers, piezo-electric actuators, light emitting electronics which include LEDs, and combinations thereof. The purpose and advantage of using standard ICs (in embodiments, CMOS, on single crystal silicon) is to have and use high quality, high performance, and high functioning circuit components that are also already commonly mass-produced with well known processes, and which provide a range of functionality and generation of data far superior to that produced by a passive means.

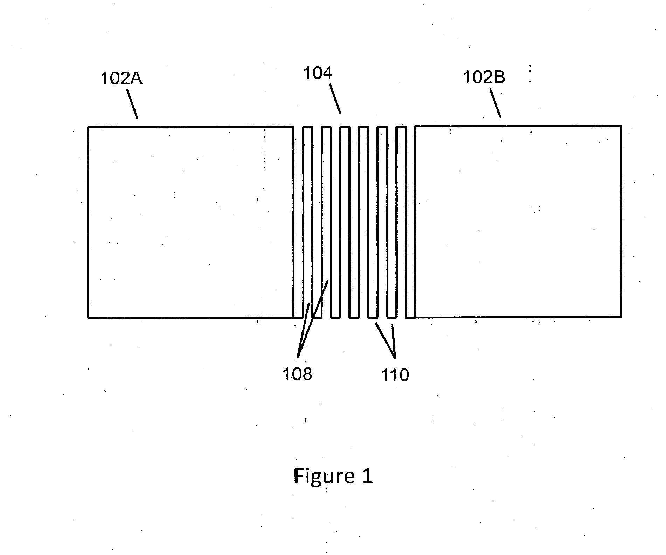

In an example, the discrete islands 102 may range from about, but not limited to, 10-100 µm in size measured on an edge or by diameter, and connecting said islands 102A-B with one or more extremely stretchable interconnects 104. The novel geometry of the interconnects 104 is what makes them extremely compliant. Each interconnect 104 is patterned and etched so that its structural form has width and thickness dimensions that may be of comparable size (such as their ratio or inverse ratio not exceeding about a factor of 10); and may be preferably equal in size. In embodiments, the dimensions may not be greater than about 5 µm (e.g. where both dimensions are about 1 µm or less). The interconnect 104 may be formed in a boustrophedonic style such that it effectively comprises long bars 108 and short bars 110 as shown in Figure 1. This unique geometry minimizes the stresses that are produced in the interconnect 104 when subsequently stretched because it has the effective form of a wire, and behaves very differently than interconnect form factors having one dimension greatly exceeding the other two (for example plates). Plate type structures primarily relieve stress only about a single axis via buckling, and withstand only a slight amount of shear stress before cracking. This invention may relieve stress about all three axes, including shears and any other stress.

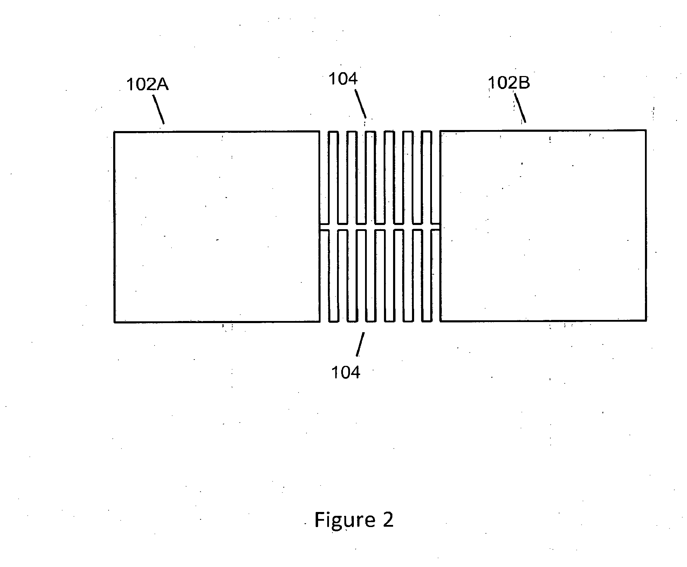

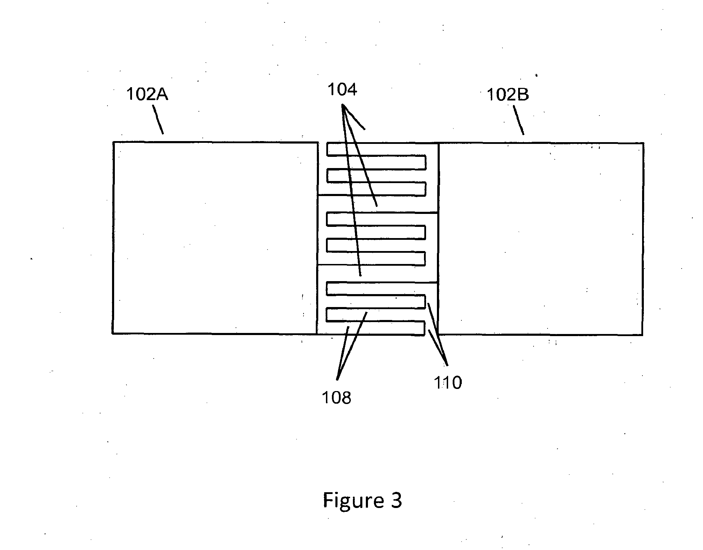

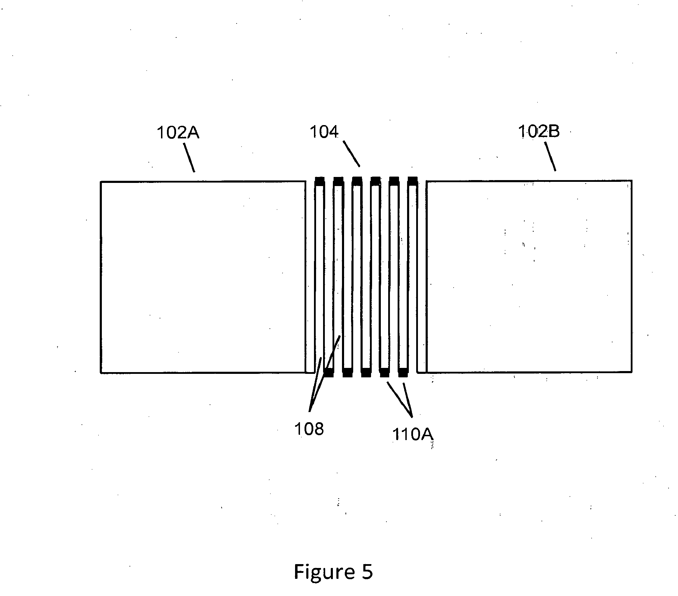

In addition, because the interconnect 104 may be formed out of rigid materials, after being stretched it may have a restorative force which helps prevent its wire-like form from getting tangled or knotted when re-compressing to the unstretched state. Another advantage of the boustrophedonic geometry is that it minimizes the initial separation distance between the islands 102A-B. This is illustrated in Figure 1. One or more interconnects 104 may be formed in various ways, as shown in Figures 2-4. The parts of the interconnect 104 where the majority of stresses build up during stretching may be the short linking bars. To minimize cracking here, the short linking bars 110A may be made several micrometers wider than the longer bars 108, as shown in Figure 5.

In embodiments, the connection point of the interconnect 104 to the device island 102 may be anywhere along the device island edge, or may be at a point on the surface of the device island 102 (in which case the interconnect may be located just above the plane of the device island).

In embodiments, device islands 102 may be made on any suitable material substrate, provided that a top membrane layer of said substrate that contains the ICs can be freed from the bulk of the substrate and transfer printed onto an elastomeric substrate.

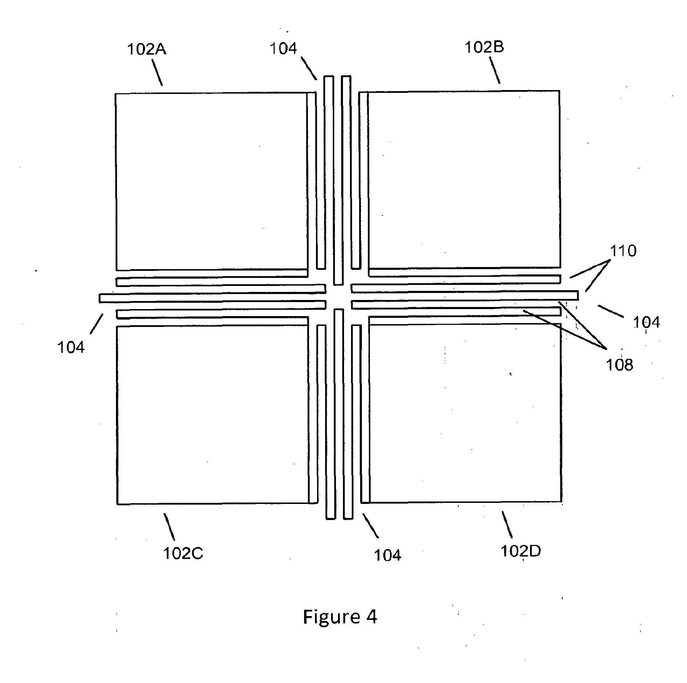

In the present invention, the interconnects 104 (as described herein) may be formed either monolithically (i.e., out of the same semiconductor material as the device islands) or may be formed out of another material. In one non-limiting example embodiment, the stretchable electronics are fabricated on a silicon-on-insulator (SOI) wafer, having a 1 µm thick top silicon layer and a 1 µm thick buried oxide layer. Devices are formed on the top silicon wafer, and arranged into a square pattern of islands 102A-D and interconnects 104 of the general form shown in Figure 4, in which the islands 102 are 100 µm on an edge, and the interconnects 104 are 1 µm wide, and the space between each long bar is 1 µm, and the interconnects 104 comprise 10 long bars 108, all about 100 µm long. The islands 102 and interconnects 104 are formed in an etching step which removes the excess silicon. The islands 102 and interconnects 104 are coated with a 1 um layer of polyimide that is patterned to only cover the islands 102 and interconnects 104. Next, the islands 102 and interconnects 104 are released in an HF etch which undercuts the underlying buried oxide. After drying, the islands 102 and interconnects 104 are transfer printed with a Polydimethylsiloxane (PDMS) stamp onto an elastomeric substrate 602. After being picked up by the transfer stamp, and prior to being placed onto the elastomeric substrate 602, the backsides of the islands 102 may be coated with a layer of polyimide (patterned to only cover the islands 102 and interconnects 104), and an additional layer of evaporated 3 nm chromium and 30 nm silicon dioxide selectively over the island regions to improve adhesion to the elastomeric substrate 602 at those locations, and not along the interconnects 102. The elastomeric substrate 602 may be PDMS or another highly compliant material. The elastomeric substrate 602 may additionally be molded or etched into the shape shown in Figure 6A-B, to further increase selective adhesion in the device island region but not the interconnect region, and to reduce the amount of material strain in the elastomeric substrate 602 that is transferred to the device islands 102. In this example, the interconnects may accommodate stretching the device islands apart by approximately up to 800 µm. In addition, the interconnects 104 of this example may be capable of accommodating lateral shear displacements of about 800 µm. In general, they may be capable of accommodating any relative displacement of the two islands such that they remain approximately within 800 µm of each other. In addition, the interconnects 104 may accommodate corkscrew type rotations of one island relative to another about any of the three axes of rotation. This feature may be limited only by the interconnects becoming entangled within each other. In any practical application, the completed stretchable device may not be so severely rotated, and the interconnect may easily accommodate rotations of up to 180°. It is noted that by increasing the number of long bars 108 used in the interconnect 104, or by increasing the length of the long bars 108, the interconnect may be able to accommodate even larger displacement strains. In embodiments, there may be no practical upper limit to the amount of displacement enabled through the present invention.

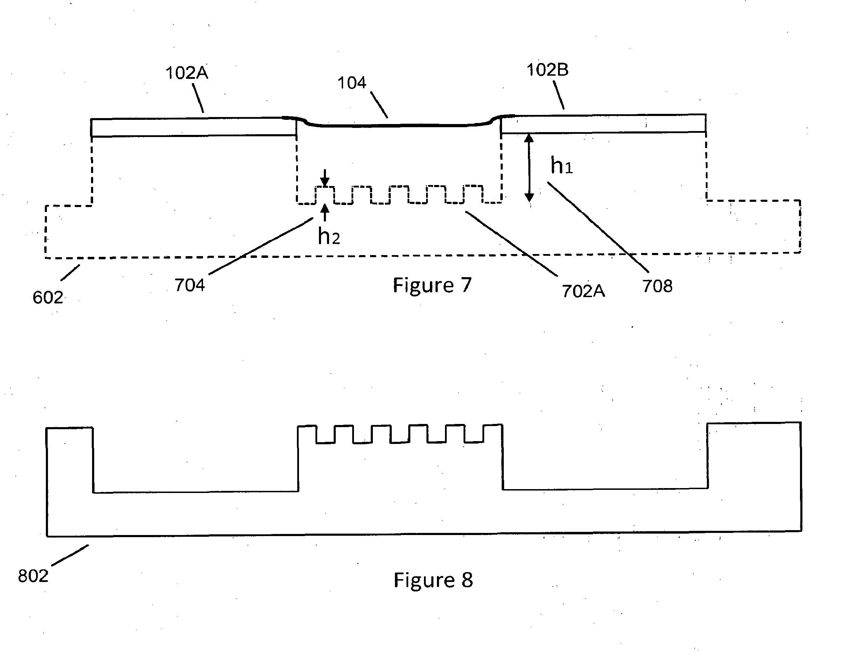

In another embodiment the elastomeric substrate 602 may comprise two layers separated by a height. The top "contact" layer contacts the device island 102 as in the embodiment illustrated in Figure 6. In addition, figure 7 shows the bottom layer 702 may be a "wavy" layer containing ripples or square waves molded into the substrate 602 during elastomer fabrication. These square waves enable additional stretching, whose extent depends on the amplitude and wavelength of the waves pattern-molded in the elastomer 602. Figure 7 shows one non-limiting layout and topology of an elastomeric substrate 602 relative to the position of the interconnects 104 and device islands 102A-B. In an example, a two layer molded substrate can be fabricated using two step process consisting of two types of negative photoresist (SU-8 50 and SU-8 2002; Microchem Corporation). The negative resists can be spin-coated on a transfer silicon wafer with spin speeds of 3000 rpm. The SU-8 50 layer can be spun on the wafer, and subsequently cured with UV radiation. Once the SU-8 50 layer has hardened, the SU-8 2002 can be spun and cured with a photo-mask and an alignment tool. In this example, the thickness of the SU-8 50 and SU-8 2002 are 40-50 µm 708 and 2-10 µm 704, respectively. The 40-50 µm thick regions of SU-8 50 contain ripples 702 of SU-8 2002 (in this instance in the form of square waves) on their surfaces. Upon curing of the SU-8 2002 layer, liquid PDMS can be poured over the SU-8 patterns to form a substrate in the shape of the SU-8 molds 802, as shown in Figure 8. The amplitude of the ripples in the SU-8 mold 802 can be varied by changing the spin speed used for spinning the thin layer of SU-8 2002. In this configuration, the interconnects 104 are free-standing. The entire substrate-device configuration can be immersed in non-cured elastomer (fluid layer) layer followed by a cured layer of PDMS to encapsulate the fluid and devices.

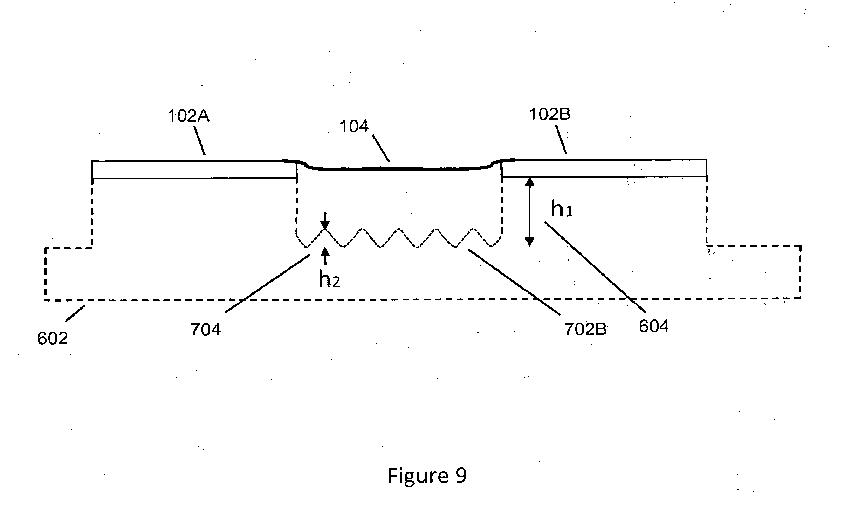

In another embodiment, the PDMS in the lower layer may be designed with periodic sinusoidal ripples 702B. In embodiments, this ripple configuration may be achieved by bonding Si nanoribbons on the surface of pre-strained PDMS in a uniform parallel pattern. The release of the prestrain in the PDMS substrate generates sinusoidal waves along the thin Si-nanoribbons (caused by buckling) and the surface of the PDMS substrate. The amplitude and wavelength of these waves 702B may depend on the extent of uniaxial pre-strain exerted on the PDMS and on the mechanical properties of the Si-nanoribbons. The wavy surface on the PDMS may be used as a transfer mold. Two-part liquid plastic solution can be poured over the wavy PDMS substrate and cured at room temperature over time (∼2 hrs). Once the plastic hardens, the plastic substrate can be peeled away from the PDMS. This new plastic transfer substrate with wavy surface features can be used to produce more PDMS substrates containing wave features. The wavy PDMS may serve as the lower layer of PDMS as in the previous embodiment. To produce a two layer PDMS structure, a top layer of PDMS can be plasma bonded to this lower layer of PDMS using oxygen plasma surface activation to produce the substrate illustrated in Figure 9.

In another embodiment, the PDMS transfer stamp is stretched after the islands 102A-B and interconnects 104 are picked up. A subsequent transfer to another elastomeric substrate 602 may place these pre-stretched devices in a configuration, which allows the new elastomeric substrate to undergo compression. The devices may be able to accommodate that compression because the interconnects are pre-stretched.

In another embodiment, the interconnects 104 are not made out of the same material as the device islands 102. In this case, the islands 102A-B are completely isolated from each other by etching, with no interconnects in between. In an example, a layer of polyimide may then be deposited, contact vias etched to various locations on the surface of the device island 102, and then metal interconnects 104 deposited and patterned into a boustrophedonic pattern, followed by another layer of polyimide. Both layers of polyimide may now be patterned and etched to leave a small border around the interconnects 104 (thereby fully encapsulating the interconnects). These interconnects may have the advantage that they are already fully encapsulated in polyimide and will not adhere as well to the elastomeric substrate as the device islands will. The other advantage is that these interconnects may not be limited to only connecting along the edge of an island. The contact via may be etched anywhere on the surface of the island 102, including near the center. This may allow for easier connections to devices, more connections than possible only along an edge, increased strain compliance, decreased strain at the contact vias, and multiple layers of interconnects made with polymer passivation layers in between, allowing even more interconnects, or allowing one device island 102A to connect to a non-neighboring device island 102B.

In another embodiment of the invention, the device islands 102 are fabricated and transfer printed onto the elastomeric substrate 602, or substrate comprising a polymeric release layer and polymeric non-release layer. After transfer printing, the interconnects 104 are formed as described above, which may be possible because they do not require any high temperature processing, and then in the latter case, the release layer is etched and the devices that are on the non-release layer, are transfer printed onto another elastomeric substrate 602. In the former case, the islands 102 may be transferred onto the elastomeric substrate using pick and place technology so that islands 102 that are initially fabricated very close to each other are spread apart when they are transfer printed. This allows the interconnects 104 to be fabricated in a pattern that resembles their stretched configuration (if desired), to allow compression.

In embodiments, the present invention may comprise a stretchable electrical interconnect 104, including an electrical interconnect 104 for connecting two electrical contacts 102A-B (e.g. device islands 102A-B), where the electrical interconnect 104 may be arranged boustrophedonicially to define rungs 108 (i.e. long bars 108) between the contacts 102A-B, and where the rungs 108 may be substantially parallel with one another and where a plurality of rungs 108 may have substantially the same length and displacement therebetween. In addition, the ratio of the length of the plurality of rungs 108 and the displacement between the plurality of rungs 108 may be large, such as at least 10:1, 100:1, 1000:1, and the like. The electrical integrity of the electrical interconnect 104 may be maintained as stretched, such as to displacements that are increased to 1000%, 10000%, 100000%, and the like during stretching. In embodiments, the rungs 108 may be substantially perpendicular to the contacts 102A-B, the interconnection 104 may have a trace width and/or inter-rung spacing ranging between 0.1-10 microns. In embodiments, the two electrical contacts 102A-B may be located on an elastomeric substrate 602, the electrical contacts 102A-B may be bonded to the substrate 602 and the interconnection 104 not bonded to the substrate 602, the electrical contacts 102A-B may be semiconductor circuits, metal contacts, and the like.

In embodiments, the present invention may comprise a stretchable electrical interconnect 104, including an electrical interconnect 104 for connecting two electrical contacts 102A-B, where the electrical interconnect 104 is arranged boustrophedonicially to define rungs 108 between the contacts 102A-B, and where the interconnect 104 maintains electrical conductivity and electrical integrity when a displacement between the contacts 102A-B is increased, such as by 1000%, 10000%, 100000%, and the like.

In embodiments, the present invention may electrically interconnect two electrical contacts 102A-B with a stretchable interconnection 104 that has the ability to twist between the two electrical contacts 102A-B by up to approximately 180 degrees while maintaining electrical integrity of the stretchable interconnection 104.

In embodiments, the present invention may be a device including a body having a stretchable surface (e.g. an elastomeric substrate 602), and a stretchable electronic circuit including (i) a first discrete operative device 102A, (ii) a second discrete operative device 102B, and (iii) a stretchable interconnect 104 connecting the first discrete operative device 102A to the second discrete operative device 102B, where the interconnect 104 may have a substantially boustrophedonic pattern and be able to maintain electrical conductivity when stretched, such as up to 1000% , 10000%, 100000%, and the like. The stretchable electronic circuit may be affixed to the stretchable surface of the body. In embodiments, the connection may be to a metal contact, to a semiconductor device, and the like. The first discrete operative device 102A, the second discrete operative device 102B, and the stretchable interconnect 104 may all be made from the same material, and that material may be a semiconductor material.

In embodiments, the present invention may attach at least two isolated electronic components (which in embodiments may be discrete operative devices) 102A-B to an elastomeric substrate 602, and arrange an electrical interconnection 104 between the components 102A-B in a boustrophedonic pattern interconnecting the two isolated electronic components 102A-B with the electrical interconnection 104. The elastomeric substrate 602 may then be stretched such that components 102A-B separate relative to one another, where the electrical interconnection 104 maintains substantially identical electrical performance characteristics that the electrical interconnection 104 had in a pre-stretched form. In embodiments, the stretching may be a translational stretching, where the separation between the isolated electronic components 102A-B increases by a percent as a result of the stretching, such as 10%, 100%, 1000%, 10000%, 100000%, and the like. The stretching may be a rotational stretching, where the rotation may be greater than a certain rotation angle, such as 90°, 180°, 270°, 360°, and the like, where the stretching may be in all three axes. In embodiments, the electrical interconnection 104 may be made from semiconducitve material. The electrical interconnection 104 may be made from the same semiconductor material as the isolated electronic components 102A-B, fabricated at the same time as the isolated electronic components 102A-B, and the like. The semiconductor material may be a single crystal semiconductor material. The electrical interconnection 104 may made of a different material than the isolated electronic components 102A-B, such as a metal. In embodiments, the interconnect material 104 may be loosely bound to the elastomeric substrate 602, not connected at all, raised above the surface of the elastomeric substrate 602, and the like. In embodiments, the at least two isolated semiconductor circuits may be fabricated on an upper surface 604 of the elastomeric substrate 602 separated by a lower surface 608 of the elastomeric substrate 602, and the electrical interconnection 104 may be fabricated at the level of the upper surface 604 of the elastomeric substrate 602. In this way, the electrical interconnection 104 may have no direct contact with the lower level 608, and thereby be substantially free from adhesion to the lower level 608 during stretching. In addition, the lower surface 608 of the elastomeric substrate 602 may include a wavy form 702, where the wavy form 704 may allow the elastomeric substrate 602 to expand during stretching.

## Claims

1. An apparatus, comprising:
a first discrete island of electronics (102A);
a second discrete island of electronics (102B); and
at least one stretchable electrical interconnect (104) to electrically connect the first and second discrete islands, said electrical interconnect arranged boustrophedonicially to define a plurality of rungs between said discrete islands, said rungs being substantially parallel with one another and having substantially the same length and displacement therebetween, **characterised in that** a ratio of said length of said plurality of said rungs and said displacement between said plurality of said rungs is at least 10:1, and
wherein the at least one stretchable electrical interconnect is formed of a rigid semiconductor material.

2. The apparatus of claim 1, wherein the at least one stretchable electrical interconnect has width and thickness dimensions of comparable size such that a ratio or inverse ratio of the width and thickness dimensions does not exceed a factor of 10.

3. The apparatus of any of the foregoing claims, wherein the at least one stretchable electrical interconnect has a trace width ranging between 0.1 micrometers to 10 micrometers.

4. The apparatus of any of the foregoing claims, wherein the at least one stretchable electrical interconnect comprises:
a plurality of long bars (108); and
a plurality of short bars (110A),
wherein the short bars are wider than the long bars.

5. The apparatus of any of the foregoing claims, wherein the at least one stretchable electrical interconnect comprises at least ten long bars (108).

6. The apparatus of any of the foregoing claims, wherein a connection point of the at least one stretchable electrical interconnect to at least one of the first and second discrete islands is along an edge or on a surface of the at least one of the first and second discrete islands.

7. The apparatus of any of the foregoing claims, including multiple layers of stretchable electrical interconnects with polymer passivation layers between layers of stretchable electrical interconnects.

8. The apparatus of any of the foregoing claims, wherein at least one of the first discrete island and the second discrete island includes at least one of:
an integrated circuit;
a physical, biological and/or chemical sensor;
an amplifier;
an A/D converter;
a D/A converter;
one or more optical collectors;
one or more electro-mechanical transducers;
one or more piezo-electric actuators; and
light emitting electronics including light emitting diodes (LEDs)

9. The apparatus of any of the foregoing claims, wherein:
the first and second discrete islands are formed of the same semiconductor material as the stretchable electrical interconnect.

10. The apparatus of any of claims 1 through 8, wherein:
the first and second discrete islands are formed of a semiconductor material.

11. The apparatus of any of the foregoing claims, wherein the at least one stretchable electronic interconnect is encapsulated in polyimide.

12. The apparatus of any of the foregoing claims, further comprising:
an elastomeric substrate (602) on which the first and second discrete islands are located,
wherein the first and second discrete islands are bonded to the substrate and the stretchable electrical interconnect is not bonded to the substrate.

13. The apparatus of claim 12, wherein:
the elastomeric substrate comprises an upper surface 604 formed by molded post regions and a lower surface 608 located between the molded post regions;
the first and second discrete device islands are disposed on the upper surface; and
the stretchable electrical interconnect is located between the molded post regions and has no direct contact with the lower surface.

14. The apparatus of claim 13, wherein the lower surface of the elastomeric substrate includes a wavy form (702, 704) to allow expansion of the elastomeric substrate.

15. The apparatus of any of claims 12 through 14, further comprising an elastomeric superstrate serving as an encapsulation layer to protect the first and second discrete islands from direct mechanical contact.

## Patentansprüche

1. Vorrichtung, umfassend:
eine erste diskrete Elektronikinsel (102A),
eine zweite diskrete Elektronikinsel (102B) und
wenigstens eine dehnbare elektrische Verbindung (104) zum elektrischen Verbinden der ersten und der zweiten diskreten Insel, wobei die genannte elektrische Verbindung boustrophedon angeordnet ist, um mehrere Zeilen zwischen den genannten diskreten Inseln zu definieren, wobei die genannten Zeilen im Wesentlichen parallel zueinander sind und im Wesentlichen die gleiche Länge und Distanz zwischen ihnen haben, **dadurch gekennzeichnet, dass** ein Verhältnis der genannten Länge der genannten mehreren der genannten Zeilen und der genannten Distanz zwischen den genannten mehreren der genannten Zeilen wenigstens 10:1 beträgt und
wobei die wenigstens eine dehnbare elektrische Verbindung aus einem starren Halbleitermaterial hergestellt ist.

2. Vorrichtung nach Anspruch 1, wobei die wenigstens eine dehnbare elektrische Verbindung Breiten- und Dickenmaße vergleichbarer Größe hat, so dass ein Verhältnis oder umgekehrtes Verhältnis des Breiten- und Dickenmaßes einen Faktor von 10 nicht übersteigt.

3. Vorrichtung nach einem der vorhergehenden Ansprüche, wobei die wenigstens eine dehnbare elektrische Verbindung eine von 0,1 Mikrometern bis 10 Mikrometern reichende Leiterbahnbreite hat.

4. Vorrichtung nach einem der vorhergehenden Ansprüche, wobei die wenigstens eine dehnbare elektrische Verbindung Folgendes aufweist:
mehrere lange Stäbe (108) und
mehrere kurze Stäbe (110A),
wobei die kurzen Stäbe breiter als die langen Stäbe sind.

5. Vorrichtung nach einem der vorhergehenden Ansprüche, wobei die wenigstens eine dehnbare elektrische Verbindung wenigstens zehn lange Stäbe (108) aufweist.

6. Vorrichtung nach einem der vorhergehenden Ansprüche, wobei ein Verbindungspunkt der wenigstens einen dehnbaren elektrischen Verbindung zu der ersten und/oder zweiten diskreten Insel sich an einem Rand entlang oder auf einer Oberfläche der ersten und/oder zweiten diskreten Insel befindet.

7. Vorrichtung nach einem der vorhergehenden Ansprüche, die mehrere Schichten aus dehnbaren elektrischen Verbindungen mit Polymerpassivierungsschichten zwischen Schichten aus dehnbaren elektrischen Verbindungen beinhaltet.

8. Vorrichtung nach einem der vorhergehenden Ansprüche, wobei die erste diskrete Insel und/oder die zweite diskrete Insel wenigstens eines der Folgenden beinhaltet:
eine integrierte Schaltung,
einen physikalischen, biologischen und/oder chemischen Sensor,
einen Verstärker,
einen A/D-Wandler,
einen D/A-Wandler,
einen oder mehrere optische Kollektoren,
einen oder mehrere elektromechanische Messwandler,
einen oder mehrere piezoelektrische Aktoren und
lichtemittierende Elektronik einschließlich lichtemittierende Dioden (LED).

9. Vorrichtung nach einem der vorhergehenden Ansprüche, wobei:
die erste und die zweite diskrete Insel aus dem gleichen Halbleitermaterial hergestellt sind wie die dehnbare elektrische Verbindung.

10. Vorrichtung nach einem der Ansprüche 1 bis 8, wobei:
die erste und die zweite diskrete Insel aus einem Halbleitermaterial hergestellt sind.

11. Vorrichtung nach einem der vorhergehenden Ansprüche, wobei die wenigstens eine dehnbare elektronische Verbindung in Polyimid gekapselt ist.

12. Vorrichtung nach einem der vorhergehenden Ansprüche, die ferner Folgendes aufweist:
ein elastomeres Substrat (602), auf dem sich die erste und die zweite diskrete Insel befinden,
wobei die erste und die zweite diskrete Insel auf das Substrat gebondet sind und die dehnbare elektrische Verbindung nicht auf das Substrat gebondet ist.

13. Vorrichtung nach Anspruch 12, wobei:
das elastomere Substrat eine obere Oberfläche 604 aufweist, die von geformten Sockelregionen gebildet wird, und eine zwischen den geformten Sockelregionen befindliche untere Oberfläche 608, aufweist,
die erste und die zweite diskrete Bauteilinsel auf der oberen Oberfläche angeordnet sind, und
die dehnbare elektrische Verbindung sich zwischen den geformten Sockelregionen befindet und keinen direkten Kontakt mit der unteren Oberfläche hat.

14. Vorrichtung nach Anspruch 13, wobei die untere Oberfläche des elastomeren Substrats eine wellige Form (702, 704) beinhaltet, um die Ausdehnung des elastomeren Substrats zuzulassen.

15. Vorrichtung nach einem der Ansprüche 12 bis 14, die ferner eine elastomere Auflage aufweist, die als eine Kapselungsschicht dient, um die erste und die zweite diskrete Insel vor direktem mechanischen Kontakt zu schützen.

## Revendications

1. Un appareil, comprenant:
un premier îlot discret de composants électroniques (102A) ;
un second îlot discret de composants électroniques (102B) ; et
au moins une interconnexion électrique étirable (104) pour connecter électriquement les premier et second îlots discrets, ladite interconnexion électrique agencée de manière boustrophédonique pour définir une pluralité de barreaux entre lesdits îlots discrets, lesdits barreaux étant essentiellement parallèles les uns avec les autres et ayant essentiellement la même longueur et le même décalage entre eux, **caractérisé en ce que** le rapport de ladite longueur de ladite pluralité desdits barreaux et ledit décalage entre ladite pluralité desdits barreaux est au moins de 10:1 ; et
dans lequel la au moins une interconnexion électrique étirable est formée dans un matériau semi-conducteur rigide.

2. L'appareil selon la revendication 1, dans lequel la au moins une interconnexion électrique étirable a des dimensions de largeur et d'épaisseur qui peuvent être de taille comparable de sorte que le rapport ou rapport inverse des dimensions de largeur et d'épaisseur ne soit pas supérieur à un facteur de 10.

3. L'appareil selon l'une quelconque des revendications précédentes, dans lequel la au moins une interconnexion électrique étirable a une largeur de trace comprise entre 0,1 micromètre et 10 micromètres.

4. L'appareil selon l'une quelconque des revendications précédentes, dans lequel la au moins une interconnexion électrique étirable comprend :
une pluralité de barres longues (108) ; et
une pluralité de barres courtes (110A)
dans lequel les barres courtes sont plus larges que les barres longues.

5. L'appareil selon l'une quelconque des revendications précédentes, dans lequel la au moins une interconnexion électrique étirable comprend au moins dix barres longues (108).

6. L'appareil selon l'une quelconque des revendications précédentes, dans lequel un point de connexion de la au moins une interconnexion électrique étirable avec au moins un des premier et second îlots discrets est le long d'un bord ou sur une surface du au moins un des premier et second îlots discrets.

7. L'appareil selon l'une quelconque des revendications précédentes, incluant des couches multiples d'interconnexions électriques étirables avec des couches de passivation polymère entre les couches d'interconnexions électriques étirables.

8. L'appareil selon l'une quelconque des revendications précédentes, dans lequel au moins un du premier îlot discret et du second îlot discret inclut au moins un de :
un circuit intégré ;
un capteur physique, biologique et/ou chimique ;
un amplificateur ;
un convertisseur A/N ;
un convertisseur N/A ;
un ou plusieurs collecteurs optiques ;
un ou plusieurs transducteurs électromécaniques ;
un ou plusieurs actionneurs piézoélectriques ; et
des composants électroniques électroluminescents incluant des diodes électroluminescentes (LED)

9. L'appareil selon l'une quelconque des revendications précédentes, dans lequel :
les premier et second îlots discrets sont formés dans le même matériau semi-conducteur que l'interconnexion électrique étirable.

10. L'appareil selon l'une quelconque des revendications 1 à 8, dans lequel :
les premier et second îlots discrets sont formés dans un matériau semi-conducteur.

11. L'appareil selon l'une quelconque des revendications précédentes, dans lequel la au moins une interconnexion électrique étirable est encapsulée dans du polyimide.

12. L'appareil selon l'une quelconque des revendications précédentes, comprenant en outre :
un substrat élastomère (602) sur lequel les premier et second îlots discrets sont situés,
dans lequel les premier et second îlots discrets sont collés au substrat et l'interconnexion électrique étirable n'est pas collée au substrat.

13. L'appareil selon la revendication 12, dans lequel :
le substrat élastomère comprend une surface supérieure 604 formée par des régions de tiges moulées et une surface inférieure 608 située entre les régions de tiges moulées ;
les premier et second îlots discrets sont agencés sur la surface supérieure ; et
l'interconnexion électrique étirable est située entre les régions de tiges moulées et n'a pas de contact direct avec la surface inférieure.

14. L'appareil selon la revendication 13, dans lequel la surface inférieure du substrat élastomère inclut une forme ondulée (702, 704) pour permettre l'expansion du substrat élastomère.

15. L'appareil selon l'une quelconque des revendications 12 à 14, comprenant en outre un superstrat élastomère servant de couche d'encapsulation pour protéger les premier et second îlots discrets contre un contact mécanique direct.
